# EUROPÄISCHE PATENTSCHRIFT

(11) **EP 2 482 770 B1**
(45) Veröffentlichungstag und Bekanntmachung des Hinweises auf die Patenterteilung: **11.03.2015**
(21) Anmeldenummer: 09778785.7
(22) Anmeldetag: 30.09.2009
(51) Int. Cl.: A61F 9/008, A61B 3/10, A61B 3/117, A61F 9/009

(54) **VORRICHTUNG FÜR DIE OPHTHALMOLOGISCHE LASERCHIRURGIE**
DEVICE FOR OPHTHALMOLOGICAL LASER SURGERY
DISPOSITIF POUR LA CHIRURGIE OPHTALMOLOGIQUE AU LASER

(43) Veröffentlichungstag der Anmeldung: 08.08.2012
(73) Patentinhaber: WaveLight GmbH, 91058 Erlangen (DE)
(72) Erfinder: RIEDEL, Peter, 90489 Nürnberg (DE); DONITZKY, Christof, 90542 Eckental (DE)
(74) Vertreter: Katérle, Axel
(86) Internationale Anmeldenummer: PCT/EP2009/007030
(87) Internationale Veröffentlichungsnummer: WO 2011/038748

(56) Entgegenhaltungen:
- EP-A2- 0 697 611
- US-A- 5 549 632
- US-A- 6 099 522
- US-A1- 2006 195 076
- IZATT J A ET AL: "MICROMETER-SCALE RESOLUTION IMAGING OF THE ANTERIOR EYE IN VIVO WITH OPTICAL COHERENCE TOMOGRAPHY" ARCHIVES OF OPHTHALMOLOGY, AMERICAN MEDICAL ASSOCIATION, US, Bd. 112, 1. Dezember 1994 (1994-12-01), Seiten 1584-1589, XP001040826 ISSN: 0003-9950

## Beschreibung

Die Erfindung betrifft eine Vorrichtung für die ophthalmologische Laserchirurgie.

Gepulste Laserstrahlung kommt bei zahlreichen Techniken der Behandlung des menschlichen Auges zum Einsatz. Bei einigen dieser Techniken wird das zu behandelnde Auge gegen ein transparentes Kontaktelement gedrückt, das mit seiner augenzugewandten Kontaktfläche eine Referenzfläche bildet, welche eine präzise Positionierung des Strahlfokus im Auge in z-Richtung ermöglichen soll. Die z-Richtung meint hierbei in Übereinstimmung mit der in der Fachwelt üblichen Notation die Ausbreitungsrichtung des Laserstrahls. Die zu dieser Richtung orthogonale Ebene wird dagegen üblicherweise als x-y-Ebene bezeichnet. Insbesondere Behandlungstechniken, die der Erzeugung von Schnitten (Inzisionen) im Augengewebe mittels fokussierter Femtosekunden-Laserstrahlung dienen (die Schnitterzeugung im menschlichen Auge mittels gepulster Femtosekunden-Laserstrahlung beruht regelmäßig auf dem Effekt des sogenannten laserinduzierten optischen Durchbruchs, der zu einer Photodisruption führt), bedienen sich häufig solcher Kontaktelemente, um damit die Position der Augenvorderfläche im Koordinatensystem der Laservorrichtung eindeutig festzulegen. Indem das Kontaktelement so gegen das Auge gedrückt wird, dass sich eine anschmiegende flächige Anlage des Auges an der augenzugewandten Kontaktfläche des Kontaktelements einstellt, gibt das Kontaktelement die z-Lage der Augenvorderfläche vor.

Eine Behandlungsform, bei der lasertechnisch ein kornealer Schnitt erzeugt wird, ist die sogenannte Fs-LASIK. Bei dieser wird mittels Femtosekunden-Laserstrahlung ein in der Fachwelt als Flap bezeichnetes anteriores Deckelscheibchen der Kornea freigeschnitten. Anschließend wird wie bei der klassischen LASIK-Technik (LASIK: Laser In Situ Keratomileusis) der noch in einem Scharnierbereich (*Hinge*) am restlichen Korneagewebe hängende Flap zur Seite geklappt und das so freigelegte Gewebe mittels UV-Laserstrahlung ablatierend bearbeitet. Eine andere Behandlungsform ist die sogenannte korneale Lentikelextraktion, bei der innerhalb des Korneagewebes ein linsenförmiges Scheibchen mittels Femtosekunden-Laserstrahlung herausgeschnitten wird. Dieses Scheibchen wird anschließend durch einen zur Augenoberfläche herausgeführten zusätzlichen Schnitt entnommen; der zusätzliche Schnitt wird entweder mittels eines Skalpells oder ebenfalls mittels Femtosekunden-Laserstrahlung erzeugt.

Die beiden erläuterten Behandlungstypen (Fs-LASIK, korneale Lentikelextraktion) sind rein beispielhaft zu verstehen. Generell ist die Erfindung bei jeglichen Behandlungstechniken anwendbar, bei denen das Auge gegen eine Kontaktfläche gedrückt wird, um hierdurch die Position der Augenvorderfläche innerhalb des Koordinatensystems der lasertechnischen Vorrichtung festzulegen.

Das Andrücken des Auges gegen die Kontaktfläche bedingt eine Verformung der Kornea. Je nach Form der Kontaktfläche kann dies zu einer zumindest lokalen Verkürzung der Vorderkammer führen, also zu einer geringeren Vorderkammertiefe. Die Vorderkammer ist der Raum zwischen der Kornea und der Linse des humanen Auges. Bei einem normalen, unverformten menschlichen Auge beträgt sie im Mittel üblicherweise etwa 2 bis 4 mm. Insbesondere bei einer Einebnung der Kornea durch Anlage an einer ebenen Kontaktfläche (Applanationsfläche) kann die Verformung der Kornea so stark sein, dass sie gefährlich nahe an die Vorderfläche der humanen Linse kommt. Eine gegenseitige Berührung der Kornearückfläche (Endothel) und der Linsenvorderfläche ist unter allen Umständen zu vermeiden. Sie könnte die korneale Endothelschicht schädigen und Trübungen in der Kornea hervorrufen.

US 2006/01 95 076 A1 zeigt eine Anordnung für die ophthalmische Laserchirurgie mit einem Bildgebungsgerät zur Aufnahme eines Bilds der inneren Kammer eines Auges und zur Bereitstellung von Informationen über die Tiefe der Innenkammer. Eine Messung von Positionen und/oder Abmessungen von Strukturen im Auge kann durchgeführt werden. Informationen des Bildgebungsgeräts werden in ein Scan-System geladen, um das lasergestützte chirurgische Verfahren zu steuern.

US 6,099,522 zeigt ebenfalls eine Anordnung für die ophthalmische Laserchirurgie. Diese Anordnung weist ein Tracking-System auf, welches Signale in Antwort auf eine Bewegung des Auges erzeugt. Ferner ist eine Unterbrechungsschaltung vorgesehen, um den Laserstrahl abzuschalten, wenn das Tracking-System ein zu verfolgendes Merkmal verliert oder im Fall von Abweichungen der Pulsenergie.

Aufgabe der Erfindung ist es, eine Vorrichtung für die ophthalmologische Laserchirurgie bereitzustellen, welche bei der Durchführung von Laserbehandlungen des menschlichen Auges, bei denen das Auge gegen eine Kontaktfläche gedrückt wird, eine hohe Sicherheit bietet, dass keine unerwünschten Beschädigungen des kornealen Endothels auftreten.

Zur Lösung dieser Aufgabe ist erfindungsgemäß eine Vorrichtung für die ophthalmologische Laserchirurgie vorgesehen, umfassend eine Kontaktfläche zur formenden Anlage eines zu behandelnden Auges, eine erste Strahlungsquelle zur Bereitstellung eines Behandlungslaserstrahls, optische Komponenten zum Richten des Behandlungslaserstrahls durch die Kontaktfläche hindurch auf das Auge und eine Messeinrichtung zur Messung der Vorderkammertiefe des an der Kontaktfläche anliegenden Auges, wobei die Messeinrichtung Messdaten bereitstellt, welche für die Vorderkammertiefe des Auges an mindestens einer Stelle desselben repräsentativ sind.

Die Erfindung lehrt, die Vorderkammertiefe zu vermessen, während das Auge gegen die Kontaktfläche gedrückt ist und die Kornea dementsprechend verformt ist. Insbesondere kann die Messung der Vorderkammertiefe vor, während oder/und nach einer Laserbehandlung wiederholt erfolgen, beispielsweise fortlaufend oder in regelmäßigen zeitlichen Abständen, um etwaige Veränderungen der Vorderkammertiefe rasch erfassen zu können. Eine Messung der Vorderkammertiefe empfiehlt sich insbesondere in der Phase, in der das Auge und die Kontaktfläche einander relativ angenähert werden, um die formende Anlage des Auges an der Kontaktfläche herzustellen. Diese relative Annäherung kann z.B. durch maschinelles oder händisches Bewegen eines die Kontaktfläche tragenden Patientenadapters oder/und einer Patientenliege erfolgen, auf welcher der Patient liegt. Zweckmäßigerweise wird im Verlauf der relativen Annäherung der Kontaktfläche und des Auges die Vorderkammertiefe mehrfach vermessen, beispielsweise bis ein vorgegebener Mindestwert für die Vorderkammertiefe erreicht wird, der auf keinen Fall unterschritten werden darf. Dieser Mindestwert sollte so gewählt sein, dass ein hinreichender Sicherheitsabstand zwischen der Kornearückfläche und der Linsenvorderfläche besteht.

Die Messeinrichtung umfasst bei einer bevorzugten Ausführungsform eine einen Messstrahl bereitstellende, zweite Strahlungsquelle, wobei die optischen Komponenten dazu ausgebildet und angeordnet sind, auch den Messstrahl durch die Kontaktfläche hindurch auf das Auge zu richten. Dies gewährleistet, dass eine Vermessung der Vorderkammertiefe in einem Zustand möglich ist, in welchem das Auge gegen die Kontaktfläche gedrückt ist.

Es kann ausreichend sein, die Vorderkammertiefe nur an einer einzigen, geeignet gewählten Stelle in der x-y-Ebene zu vermessen, beispielsweise am oder zumindest in der Nähe eines Applanationszentrums. Für eine erhöhte Sicherheit kann es aber vorteilhaft sein, wenn die Messeinrichtung dazu ausgebildet ist, die Vorderkammertiefe an verschiedenen Stellen des Auges zu messen. Beispielsweise kann die Messeinrichtung die Vorderkammertiefe an einer Vielzahl vorgegebener Messpunkte messen. Diese Messpunkte können beispielsweise einen zentralen Messpunkt sowie eine Mehrzahl in einem Kreis oder in mehreren konzentrischen Kreisen um den zentralen Messpunkt herum verteilter peripherer Messpunkte umfassen. Alternativ ist auch eine scannende Vermessung der Vorderkammertiefe vorstellbar, bei welcher die Messeinrichtung zumindest einen vorbestimmten Messbereich des Auges mit einer Vielzahl eng nebeneinander liegender Abtastpunkte abtastet und an jedem dieser Abtastpunkte die Vorderkammertiefe misst. Eine solche abtastende Vermessung der Vorderkammertiefe gestattet eine hohe Auflösung und sozusagen eine flächige Kartierung der Vorderkammertiefe.

Vorzugsweise ist mit der Messeinrichtung eine elektronische Auswerte- und Steueranordnung verbunden, welche dazu eingerichtet ist, die durch die Messdaten repräsentierte Vorderkammertiefe auf das Unterschreiten mindestens eines vorbestimmten Wertes zu prüfen und abhängig vom Unterschreiten des vorbestimmten Werts eine vorbestimmte Aktion zu bewirken. Dies ermöglicht eine automatische Überwachung der Vorderkammertiefe und die automatische Einleitung geeigneter Aktionen, sollte die Vorderkammertiefe den vorbestimmten Wert unterschreiten. Es versteht sich, dass mehrere verschiedene vorbestimmte Grenzwerte festgelegt sein können, die sozusagen einen unterschiedlichen Gefährdungsgrad darstellen. Je geringer der Abstand zwischen der Kornearückfläche und der Linsenvorderfläche wird, umso eindringlicher oder massiver kann die von der Auswerte- und Steueranordnung bewirkte automatische Reaktion sein.

Beispielsweise kann die Auswerte- und Steueranordnung dazu eingerichtet sein, bei Unterschreiten des vorbestimmten Werts die Ausgabe eines optischen oder/und akustischen Warnhinweises zu bewirken.

Alternativ oder zusätzlich kann die Auswerte- und Steueranordnung dazu eingerichtet sein, bei Unterschreiten des vorbestimmten Werts mindestens eine steuerbare Komponente im Sinne eines Stopps einer relativen Annäherungsbewegung zwischen der Kontaktfläche und dem Auge oder im Sinne einer relativen Auseinanderbewegung der Kontaktfläche und des Auges zu steuern. Die steuerbare Komponente kann beispielsweise eine Evakuierungspumpe sein, welche einen Unterdruck erzeugt, mittels dessen die Kontaktfläche an dem Auge gehalten wird oder/und ein die Kontaktfläche tragender Patientenadapter an einem auf das Auge aufgesetzten Saugring gehalten wird. Durch Verringerung oder sogar vollständige Wegnahme des Unterdrucks kann der Anlagedruck des Auges an der Kontaktfläche verringert werden. Unter Umständen kann sich die Kontaktfläche sogar von dem Auge lösen. In jedem Fall kann auf diese Weise die Kornearückfläche aus einer möglicherweise gefährlichen Nahlage zur Linsenvorderfläche weggebracht werden.

Darüber hinaus ist es alternativ oder zusätzlich möglich, dass die Auswerte- und Steueranordnung dazu eingerichtet ist, die Abgabe des Behandlungslaserstrahls abhängig davon zu gestatten, dass die durch die Messdaten repräsentierte Vorderkammertiefe den vorbestimmten Wert nicht unterschreitet. Umgekehrt kann die Auswerte- und Steueranordnung den Behandlungslaserstrahl abschalten , sobald die gemessene Vorderkammertiefe den vorbestimmten Wert unterschreitet.

Die Messeinrichtung kann ein optisches Interferometer umfassen, welches dazu eingerichtet ist, den Messstrahl und einen vom Auge durch die Kontaktfläche hindurch zurückkommenden Reflex in Interferenz zu bringen. Beispielsweise kann die Messeinrichtung eine OLCR-Messeinrichtung sein, also nach dem Prinzip der optischen Kurzkohärenz-Reflektometrie arbeiten. OLCR steht für Optical Low Coherence Reflectometry.

Ein die Kontaktfläche bildendes transparentes Kontaktelement kann entweder als Applanationsplatte oder als Kontaktglas mit nichtebener Anlagefläche für das Auge ausgebildet sein. Unter einer Applanationsplatte wird hierbei ein Kontaktelement verstanden, welches auf seiner augenzugewandten Plattenseite eine ebene Anlagefläche für die Augenvorderseite aufweist und deshalb eine Einebnung der Kornea gestattet. Auf seiner augenabgewandten Plattenseite kann die Applanationsplatte gleichermaßen eben sein; sie kann dort aber auch konkav oder konvex gekrümmt sein. Unter einem Kontaktglas wird dagegen ein solches Kontaktelement verstanden, welches auf seiner augenzugewandten Seite eine nicht ebene Anlagefläche für die Augenvorderseite aufweist. In der Regel wird diese Anlagefläche konkav gekrümmt sein.

Die Applanationsplatte bzw. das Kontaktglas kann beispielsweise an einem mit einem Fokussierobjektiv der Vorrichtung gekoppelten Patientenadapter gehalten sein.

Die Pulsdauer des Behandlungslaserstrahls liegt vorzugsweise im Femtosekundenbereich.

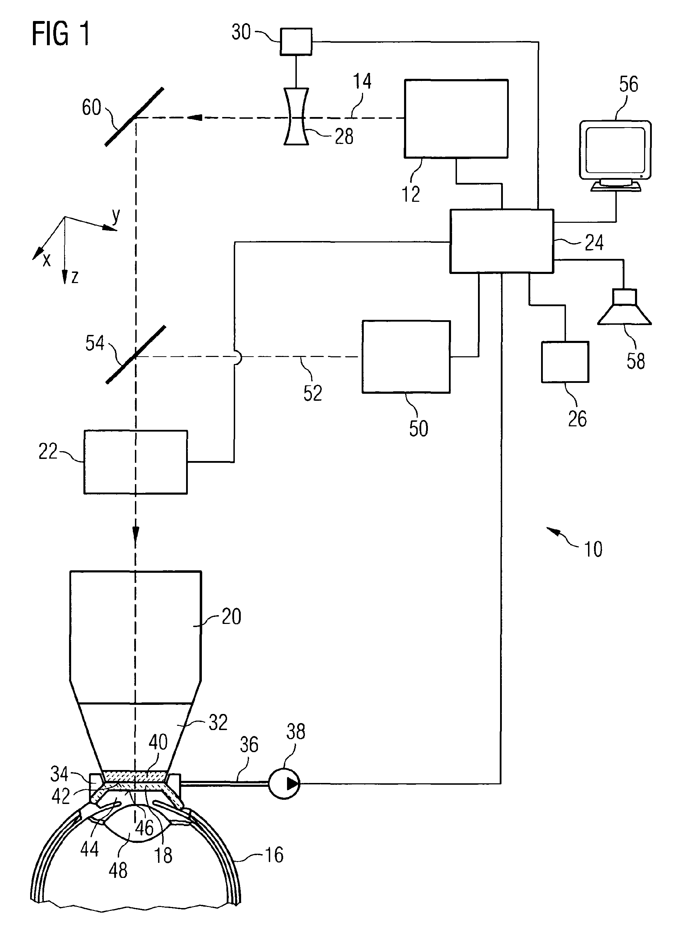

Die Erfindung wird nachfolgend anhand der beigefügten Zeichnung weiter erläutert. Deren einzige Fig. 1 stellt in stark schematisierter Form ein Ausführungsbeispiel einer Vorrichtung für die ophthalmologische Laserchirurgie dar. Die Vorrichtung ist allgemein mit 10 bezeichnet. Sie weist einen Fs-Laser 12 auf, welcher einen gepulsten Laserstrahl 14 mit Pulsdauern im Bereich von Femtosekunden abgibt. Der Laserstrahl 14 dient zur Behandlung eines humanen Auges 16 und dort beispielsweise einer Kornea 18. Insbesondere dient er zur Erzeugung von Schnitten in der Kornea 18, wobei der Schnitt durch eine Aneinanderreihung von intrakornealen Photodisruptionen entsteht, die im Strahlfokus durch den Effekt des laserinduzierten optischen Durchbruchs hervorgerufen werden.

Im Strahlengang des Laserstrahls 14 sind verschiedene optische Komponenten zur Führung und Formung des Laserstrahls 14 angeordnet. Insbesondere umfassen diese Komponenten ein Fokussierobjektiv 20 (beispielsweise ein F-Theta-Objektiv) sowie einen dem Objektiv 20 vorgeschalteten Scanner 22 mittels dessen der von dem Laser 12 abgegebene Laserstrahl 14 in einer zum Strahlengang des Laserstrahls orthogonalen Ebene (x-y-Ebene) nach Maßgabe eines für das Auge 16 ermittelten Behandlungsprofils ablenkbar ist. Ein eingezeichnetes Koordinatensystem veranschaulicht diese Ebene sowie eine durch die Richtung des Laserstrahls 14 vorgegebene z-Achse. Der Scanner 22 ist beispielsweise in an sich bekannter Weise aus einem Paar galvanometrisch gesteuerter Ablenkspiegel aufgebaut, welche jeweils für die Strahlablenkung in Richtung einer der die x-y-Ebene aufspannenden Achsen verantwortlich sind. Eine elektronische Auswerte- und Steuereinheit 24 steuert den Scanner 22 nach Maßgabe eines in einem Speicher 26 gespeicherten Steuerprogramms, welches ein in dem Auge 16 zu erzeugendes Schnittprofil repräsentiert. Das Schnittprofil ist dabei dargestellt durch die Koordinaten eines dreidimensionalen Musters von Abtastpunkten, an denen jeweils eine Photodisruption zu bewirken ist.

Des weiteren umfassen die erwähnten optischen Komponenten mindestens ein steuerbares optisches Element zur z-Verstellung des Strahlfokus des Laserstrahls 14. Im gezeigten Beispielfall ist dieses optische Element 28 von einer Linse (konkret einer Zerstreuungslinse) gebildet. Zur Steuerung der Linse 28 dient ein geeigneter Aktuator 30, der seinerseits durch die Auswerte- und Steuereinheit 24 gesteuert ist. Beispielsweise kann die Linse 28 mechanisch längs des Strahlengangs des Laserstrahls 14 verfahrbar sein. Alternativ ist es vorstellbar, eine steuerbare Flüssiglinse variabler Brechkraft zu verwenden. Bei unveränderter z-Position und auch ansonsten unveränderter Einstellung des Fokussierobjektivs 20 lässt sich durch Verfahren einer längsverstellbaren Linse oder durch Brechkraftvariation einer Flüssiglinse eine z-Verlagerung des Strahlfokus erreichen. Es versteht sich, dass zur z-Verstellung des Strahlfokus auch andere Komponenten vorstellbar sind, etwa ein verformbarer Spiegel. Wegen seiner vergleichsweise höheren Trägheit ist es zweckmäßig, mit dem Fokussierobjektiv 20 nur eine anfängliche Grundeinstellung des Strahlfokus (d.h. Fokussierung auf eine vorgegebene z-Referenzposition) vorzunehmen, aber die durch das Schnittprofil vorgegebenen z-Verlagerungen des Strahlfokus durch eine außerhalb des Fokussierobjektivs 20 angeordnete Komponente mit kürzerer Reaktionsgeschwindigkeit zu bewerkstelligen. Eine solche Komponente kürzerer Reaktionsgeschwindigkeit ist beispielsweise die Linse 28.

Auf der Seite des Strahlaustritts ist das Fokussierobjektiv 20 mit einem Patientenadapter 32 gekoppelt, welcher zur Herstellung einer mechanischen Kopplung zwischen dem Auge 16 und dem Fokussierobjektiv 20 dient. Der Patientenadapter 32 besitzt eine geeignete mechanische Schnittstelle zur Ankopplung an einen Saugring 34, welcher zu Beginn der Behandlung auf das Auge 16 aufgesetzt und dort durch Saugkraft fixiert wird. Dementsprechend ist der Saugring 34 über eine Evakuierungsleitung 36 mit einer Evakuierungspumpe 38 verbunden, welche durch die elektronische Auswerte- und Steuereinheit 24 steuerbar ist. Nach Aufsetzen des Saugrings 34 auf das Auge 16 erfolgt eine relative Annäherung des Auges 16 und des Patientenadapters 32, bis der Saugring 34 und der Patientenadapter 32 ordnungsgemäß aneinandergekoppelt sind. Bezüglich der wechselseitigen Ankopplung des Saugrings 34 und des Patientenadapters 32 kann beispielsweise auf WO 2010/022 745 A1 verwiesen werden.

Der Patientenadapter 32 dient als Träger für ein transparentes Kontaktelement 40, welches im gezeigten Beispielfall als planparallele Applanationsplatte ausgebildet ist. Der Patientenadapter 32 umfasst beispielsweise einen Kegelhülsenkörper, an dessen schmälerem (in der Zeichnung unterem) Hülsenende die Applanationsplatte 34 angeordnet ist. Im Bereich des breiteren (in der Zeichnung oberen) Hülsenendes ist der Patientenadapter 32 dagegen an dem Fokussierobjektiv 20 angesetzt und besitzt dort geeignete Formationen, die eine gewünschtenfalls lösbare Fixierung des Patientenadapters 32 an dem Fokussierobjektiv 20 gestatten.

Weil sie während der Behandlung in Kontakt mit dem Auge 18 gelangt, ist die Applanationsplatte 40 ein unter dem Gesichtspunkt der Hygiene kritischer Artikel, der deshalb zweckmäßigerweise nach jeder Behandlung auszuwechseln ist. Hierzu kann die Applanationsplatte 40 auswechselbar an dem Patientenadapter 32 angebracht sein. Alternativ kann der Patientenadapter 32 zusammen mit der Applanationsplatte 40 eine Wegwerfeinheit oder zumindest eine für den Einmalgebrauch bestimmte und dann für die weitere Verwendung wieder zu sterilisierende Einheit bilden. In diesem Fall kann die Applanationsplatte 40 unlösbar mit dem Patientenadapter 32 verbunden sein.

Jedenfalls bildet die augenzugewandte Unterseite der Applanationsplatte 40 eine ebene Kontaktfläche 42, gegen welche das Auge 16 zu drücken ist. Dies bewirkt eine Einebnung der Augenvorderfläche (allgemein eine Verformung der Kornea 18 des Auges 16). Die Einebnung der Augenvorderfläche bewirkt, dass zumindest in dem eingeebneten Bereich die Tiefe der mit 44 bezeichneten Augenvorderkammer abnimmt; die Kornearückfläche - bezeichnet mit 46 - nähert sich dort der Vorderfläche der mit 48 bezeichneten humanen Linse an.

Damit die Kornearückfläche 46 der Linsenvorderfläche nicht gefährlich nahe kommt, weist die laserchirurgische Vorrichtung 10 eine kohärenzoptische interferometrische Messeinrichtung 50 auf, bei der es sich vorzugsweise um eine OLCR-Messeinrichtung handelt. Die Messeinrichtung 50 sendet einen Messstrahl 52 aus, der mittels eines unbeweglich angeordneten, halbdurchlässigen Umlenkspiegels 54 in den Strahlengang des Laserstrahls 14 eingekoppelt wird. Der Messstrahl 52 durchläuft das Fokussierobjektiv 20, den Patientenadapter 32 sowie die Applanationsplatte 40 und trifft auf das Auge 16. Der Einfall des Messstrahls 52 auf das Auge bewirkt Reflexionen. Dieser gelangt auf demselben Weg zur Messeinrichtung 50 zurück, den der Messstrahl 52 genommen hat. In einem in der Messeinrichtung 50 enthaltenen, nicht näher dargestellten Interferometer wird der Messstrahl 52 mit dem zurückkommenden Reflexstrahl in Interferenz gebracht. Aus den diesbezüglich gewonnenen Interferenzmessdaten kann die z-Abmessung der Vorderkammer 44 ermittelt werden. Die Auswerte- und Steuereinheit 24 erhält die Interferenzmessdaten von der Messeinrichtung 50 und berechnet hieraus die Vorderkammertiefe an derjenigen Stelle in der x-y-Ebene, wo der Messstrahl 52 auftraf.

Im gezeigten Beispielfall durchläuft der von der Messeinrichtung 50 ausgesendete Messstrahl 52 auch den Scanner 22. Dies ermöglicht es, die x-y-Ablenkfunktion des Scanners 22 auch für den Messstrahl 52 zu nutzen. Es ist so eine Messung der Vorderkammertiefe an unterschiedlichen Stellen entlang der x-y-Ebene möglich. Dies gewährleistet eine hohe Sicherheit, diejenige Stelle oder denjenigen Bereich messtechnisch zu erfassen, an dem die Vorderkammertiefe am kleinsten ist. Beispielsweise kann die Messung der Vorderkammertiefe nach Maßgabe eines Musters durchgeführt werden, welches einen zentralen Messpunkt sowie weitere Messpunkte vorsieht, die in einem oder mehreren konzentrischen Kreisen um den zentralen Messpunkt herum verteilt liegen. Die hierfür nötige Ortssteuerung des Messstrahls in der x-y-Ebene kann zweckmäßigerweise mit dem Scanner 22 erreicht werden.

In einer Ausgestaltung kann der Scanner 22 ein Spiegelpaar oder eine nach einer anderen Ablenktechnik arbeitende Ablenkeinheit enthalten, das bzw. die gemeinsam zur x-y-Ablenkung des Laserstrahls 14 und des Messstrahls 52 genutzt wird. In einer anderen Ausgestaltung kann der Scanner 22 gesonderte Spiegelpaare oder allgemein gesonderte Ablenkeinheiten enthalten, von denen die eine zur x-y-Ablenkung des Laserstrahls 14 und die andere zur x-y-Ablenkung des Messstrahls 52 verwendet wird. Die Ablenkeinheit für den Messstrahl 52 könnte beispielsweise mit kleineren, schneller bewegbaren Spiegeln ausgestattet sein als die Ablenkeinheit für den Laserstrahl 14. In noch einer anderen Ausgestaltung kann eine Ablenkeinheit für den Messstrahl 52 in demjenigen Teil des Strahlengangs des Messstrahls angeordnet sein, der vor dem Umlenkspiegel 54 liegt.

Die Ermittlung der Vorderkammertiefe kann beispielsweise anhand des Abstands bestimmter Signalspitzen in einem von der Messeinrichtung 50 erzeugten Interferenzmesssignal erfolgen. Ein solches Interferenzmesssignal kann deutlich hervortretende Signalspitzen zeigen, die durch Reflektion des Messstrahls 52 an den verschiedenen Grenzflächen entstehen, auf die der Messstrahl 52 trifft. Eine solche Grenzfläche ist die augenabgewandte Vorderseite der Applanationsplatte 40, eine weitere Grenzfläche ist die auf der augenzugewandten Rückseite der Applanationsplatte 40 gebildete Kontaktfläche 42, und noch weitere Grenzflächen sind die Kornearückfläche 46 sowie die Vorderfläche der Linse 48. Der wechselseitige Abstand der Signalspitzen ist ein Maß für den z-Abstand der betreffenden Grenzflächen. Deshalb kann die Auswerte- und Steuereinheit 24 aus dem Abstand der Signalspitzen, die durch Reflektion des Messstrahls 52 an der Kornearückfläche und der Linsenvorderfläche entstehen, leicht die Vorderkammertiefe an der betreffenden Stelle ermitteln.

Die Auswerte- und Steuereinheit 24 überwacht die Vorderkammertiefe des Auges 16, um rechtzeitig geeignete Gegenmaßnahmen zu bewirken, sollte die Kornearückfläche 46 der Linsenvorderfläche gefährlich nahe kommen. Vorzugsweise überwacht die Auswerte- und Steuereinheit 24 die Vorderkammertiefe individuell an jedem Messpunkt, wo Messungen der Vorderkammertiefe erfolgen. Sollte an einem der Messpunkte der gemessene Wert der Vorderkammertiefe einen vorbestimmten Mindestwert (Grenzwert) erreichen, der nicht unterschritten werden soll, kann die Auswerte- und Steuereinheit 24 beispielsweise die Unterdruckversorgung des Saugrings 24 durch die Pumpe 38 unterbrechen, so dass der Patientenadapter 32 sich zumindest teilweise von dem Saugring 34 lösen kann und der Druck der Applanationsplatte 40 auf die Kornea 18 geringer wird. Alternativ oder zusätzlich kann die Auswerte- und Steuereinheit 24 einen optischen Warnhinweis auf einem Bildschirm 56 oder einem anderen geeigneten optischen Ausgabemittel oder/und einen akustischen Warnhinweis über einen Lautsprecher 58 ausgeben. Zweckmäßigerweise erfolgt die Überwachung der Vorderkammertiefe schon während des Ankoppelvorgangs des Patientenadapters 32 an den Saugring 34, zumindest jedenfalls unmittelbar nach Abschluss dieses Ankoppelvorgangs. Auf diese Weise kann frühzeitig erkannt werden, ob die Kornearückfläche 46 der Linse 48 gefährlich nahe kommt. Der erwähnte Grenzwert, ab dem die Auswerte- und Steuereinheit 24 Gegenmaßnahmen einleitet, kann beispielsweise einer Resttiefe der Vorderkammer von etwa 0,5 mm entsprechen.

Mit dem Bezugszeichen 60 ist ein weiterer unbeweglicher Umlenkspiegel bezeichnet, welcher der Führung des Behandlungslaserstrahls 14 dient.

## Patentansprüche

1. Vorrichtung (10) für die ophthalmologische Laserchirurgie, umfassend
- eine Kontaktfläche (42) zur formenden Anlage eines zu behandelnden Auges (16),
- eine erste Strahlungsquelle (12) zur Bereitstellung eines Behandlungslaserstrahls (14),
- optische Komponenten (20, 22, 28, 60) zum Richten des Behandlungslaserstrahls durch die Kontaktfläche hindurch auf das Auge,
- eine Messeinrichtung (50) zur Messung der Vorderkammertiefe des an der Kontaktfläche anliegenden Auges, wobei die Messeinrichtung Messdaten bereitstellt, welche für die Vorderkammertiefe des Auges an mindestens einer Stelle desselben repräsentativ sind, und
- eine mit der Messeinrichtung (50) verbundene elektronische Auswerte- und Steueranordnung (24), die dazu eingerichtet ist, die durch die Messdaten repräsentierte Vorderkammertiefe auf das Unterschreiten mindestens eines vorbestimmten Werts zu prüfen und abhängig vom Unterschreiten des vorbestimmten Werts eine vorbestimmte Aktion zu bewirken,
**dadurch gekennzeichnet, dass** die Auswerte- und Steueranordnung (24) ferner dazu eingerichtet ist, bei Unterschreiten des vorbestimmten Werts mindestens eine steuerbare Komponente (38) der Vorrichtung im Sinne eines Stopps einer relativen Annäherungsbewegung zwischen der Kontaktfläche (42) und dem Auge (16) oder im Sinne einer relativen Auseinanderbewegung der Kontaktfläche und des Auges zu steuern.

2. Vorrichtung nach Anspruch 1, wobei die Messeinrichtung (50) eine einen Messstrahl (52) bereitstellende zweite Strahlungsquelle umfasst und die optischen Komponenten dazu ausgebildet und angeordnet sind, auch den Messstrahl durch die Kontaktfläche (42) hindurch auf das Auge zu richten.

3. Vorrichtung nach Anspruch 1 oder 2, wobei die Messeinrichtung (50) dazu ausgebildet ist, die Vorderkammertiefe an verschiedenen Stellen des Auges zu messen.

4. Vorrichtung nach einem der vorhergehenden Ansprüche, wobei die Auswerte- und Steueranordnung (24) dazu eingerichtet ist, bei Unterschreiten des vorbestimmten Werts die Ausgabe eines optischen oder/und akustischen Warnhinweises zu bewirken.

5. Vorrichtung nach einem der vorhergehenden Ansprüche, wobei die Auswerte- und Steueranordnung (24) dazu eingerichtet ist, die Abgabe des Behandlungslaserstrahls (14) abhängig davon zu gestatten, dass die durch die Messdaten repräsentierte Vorderkammertiefe den vorbestimmten Wert nicht unterschreitet.

6. Vorrichtung nach einem der Ansprüche 2 bis 5, wobei die Messeinrichtung (50) ein optisches Interferometer umfasst, welches dazu eingerichtet ist, den Messstrahl (52) und einen vom Auge (16) durch die Kontaktfläche (42) hindurch zurückkommenden Reflex in Interferenz zu bringen.

7. Vorrichtung nach Anspruch 6, wobei die Messeinrichtung (50) nach dem Prinzip der optischen Kurzkohärenz-Reflektometrie arbeitet.

8. Vorrichtung nach einem der vorhergehenden Ansprüche, wobei die Kontaktfläche (42) von einem transparenten Kontaktelement gebildet ist, welches als Applanationsplatte (40) oder als Kontaktglas mit nichtebener Anlagefläche für das Auge ausgebildet ist.

9. Vorrichtung nach Anspruch 8, wobei die Applanationsplatte (40) bzw. das Kontaktglas an einem mit einem Fokussierobjektiv (20) der Vorrichtung gekoppelten Patientenadapter gehalten ist.

10. Vorrichtung nach einem der vorhergehenden Ansprüche, wobei die Pulsdauer des Behandlungslaserstrahls (14) im Femtosekundenbereich liegt.

## Claims

1. An apparatus (10) for ophthalmic surgery, comprising:
- a contact surface (42) for the shaping abutment of an eye (16) to be treated,
- a first radiation source (12) for providing a treatment laser beam (14),
- optical components (20, 22, 28, 60) for directing the treatment laser beam through the contact surface onto the eye,
- a measuring device (50) for measuring the depth of the anterior chamber of the eye bearing against the contact surface, wherein the measuring device provides measured data representative of the depth of the anterior chamber of the eye at at least one point of the same, and
- an electronic evaluating and control arrangement (24) connected to the measuring device (50), which is adapted to examine the depth of the anterior chamber represented by the measured data for falling below at least one predetermined value and, depending on falling below the predetermined value, to bring about a predetermined action,
**characterised in that** the electronic evaluating and control arrangement (24) is further adapted to control at least one controllable component (38) of the apparatus in the sense of a halt of a relative approaching movement between the contact surface (42) and the eye (16) or in the sense of a relative movement apart of the contact surface and the eye, in the case of falling below the predetermined value.

2. The apparatus according to Claim 1, wherein the measuring device (50) includes a second radiation source providing a measuring beam and the optical components are designed and arranged to direct also the measuring beam through the contact surface (42) onto the eye.

3. The apparatus according to Claim 1 or 2, wherein the measuring device (50) is adapted to measure the depth of the anterior chamber at different points of the eye.

4. The apparatus according to one of the previous claims, wherein the evaluating and control arrangement (24) is adapted to bring about an output of a visual and/or acoustic warning indication in the case of falling below the predetermined value.

5. The apparatus according to one of the previous claims, wherein the evaluating and control arrangement (24) is adapted to permit the emission of the treatment laser beam (14) depending on the depth of the anterior chamber represented by the measured data not falling below the predetermined value.

6. The apparatus according to one of Claims 2 to 5, wherein the measuring device (50) includes an optical interferometer which is adapted to cause interference between the measuring beam (52) and a reflection returning from the eye through the contact surface (42).

7. The apparatus according to one of Claim 6, wherein the measuring device (50) operates in accordance with the principle of short coherence reflectometry.

8. The apparatus according to one of the previous claims, wherein the contact surface (42) is formed by a transparent contact element designed as an applanation plate (40) or a contact lens with a non-planar abutment surface for the eye.

9. The apparatus according to Claim 8, wherein the applanation plate (40) or the contact lens is supported on a patient adapter which is coupled to a focusing objective (20) of the apparatus.

10. The apparatus according to one of the previous claims, wherein the pulse duration of the treatment laser beam (14) is within the femtosecond range.

## Revendications

1. Dispositif (10) pour la chirurgie ophtalmologique par laser, comprenant
- une surface de contact (42) pour l'appui moulant d'un oeil (16) à traiter,
- une première source de rayonnement (12) destinée à fournir un faisceau laser de traitement (14),
- des composants optiques (20, 22, 28, 60) pour diriger le faisceau laser de traitement sur l'oeil à travers la surface de contact,
- un dispositif de mesure (50) pour mesurer la profondeur de la chambre antérieure de l'oeil appliqué sur la surface de contact, le dispositif de mesure fournissant des données de mesure qui sont représentatives de la profondeur de la chambre antérieure de l'oeil en au moins un point de celui-ci, et
- un dispositif électronique d'évaluation et de commande (24) relié au dispositif de mesure (50), qui est conçu pour contrôler si la profondeur de la chambre antérieure représentée par les données de mesure passe au-dessous d'au moins une valeur prédéterminée et, en fonction du sous-passement de la valeur prédéterminée, produire une action prédéterminée,
**caractérisé en ce que** le dispositif d'évaluation et de commande (24) est en outre conçu, en cas de sous-passement de la valeur prédéterminée, pour commander au moins un composant commandable (38) dudit dispositif dans le sens d'un arrêt d'un mouvement de rapprochement relatif entre la surface de contact (42) et l'oeil (16) ou dans le sens d'un mouvement d'éloignement relatif de la surface de contact et de l'oeil.

2. Dispositif selon la revendication 1, dans lequel le dispositif de mesure (50) comprend une deuxième source de rayonnement destinée à fournir un faisceau de mesure (52) et les composants optiques sont configurés et disposés pour diriger également le faisceau de mesure sur l'oeil à travers la surface de contact (42).

3. Dispositif selon la revendication 1 ou 2, dans lequel le dispositif de mesure (50) est conçu pour mesurer la profondeur de la chambre antérieure en différents points de l'oeil.

4. Dispositif selon l'une des revendications précédentes, dans lequel le dispositif d'évaluation et de commande (24) est conçu pour émettre un signal d'avertissement optique et/ou acoustique en cas de sous-passement de la valeur prédéterminée.

5. Dispositif selon l'une des revendications précédentes, dans lequel le dispositif d'évaluation et de commande (24) est conçu pour permettre l'émission du faisceau laser de traitement (14) en fonction du fait que la profondeur de la chambre antérieure représentée par les données de mesure ne passe pas au-dessous de la valeur prédéterminée.

6. Dispositif selon l'une des revendications 2 à 5, dans lequel le dispositif de mesure (50) comprend un interféromètre optique qui est conçu pour faire interférer le faisceau de mesure (52) et un reflet revenant de l'oeil (16) à travers la surface de contact (42).

7. Dispositif selon la revendication 6, dans lequel le dispositif de mesure (50) fonctionne selon le principe de la réflectométrie optique à cohérence courte.

8. Dispositif selon l'une des revendications précédentes, dans lequel la surface de contact (42) est formée par un élément de contact transparent qui est réalisé sous la forme d'une plaque d'aplanation (40) ou d'un verre de contact à surface d'appui non plane pour l'oeil.

9. Dispositif selon la revendication 8, dans lequel la plaque d'aplanation (40) ou le verre de contact est maintenu(e) sur un adaptateur patient couplé à un objectif de focalisation (20) du dispositif.

10. Dispositif selon l'une des revendications précédentes, dans lequel la durée d'impulsion du faisceau laser de traitement (14) se situe dans le domaine des femto-secondes.
